# EUROPEAN PATENT APPLICATION

(11) **EP 2 154 137 A1**
(43) Date of publication of application: **17.02.2010**
(21) Application number: 08161718.5
(22) Date of filing: 04.08.2008
(51) Int. Cl.: C07D 471/04, A61K 31/437, A61P 31/04

(54) **Crystalline form of moxifloxacin base**

(71) Applicant: Chemo Ibérica, S.A., 08028 Barcelona (ES)
(72) Inventor: Villasante Prieto, Javier, 28007 Madrid (ES); Cosme Gomez, Antonio, 28813 Torres de Alameda (Madrid) (ES); Fau de Casa-Juana Munõz, Miguel, 28033 Madrid (ES); Martìnez del Amo, Javier, 28810 Villalbilla (Madrid) (ES)
(74) Representative: Barlocci, Anna

(57) **Abstract**

Crystalline form of moxifloxacin base of formula (I), a process for its preparation, which comprises the following steps: a) dissolving a suspension of moxifloxacin in an organic solvent which is able to be removed from water, by means of heating to the reflux temperature; b) adding water and cooling to a temperature ranging between 20°C to 40°C; c) removing the organic solvent; d) seeding with moxifloxacin base sesquihydrate; and e) isolating the crystalline form obtained. It is useful for the preparation of pharmaceutical compositions for use as an antibacterial agent.

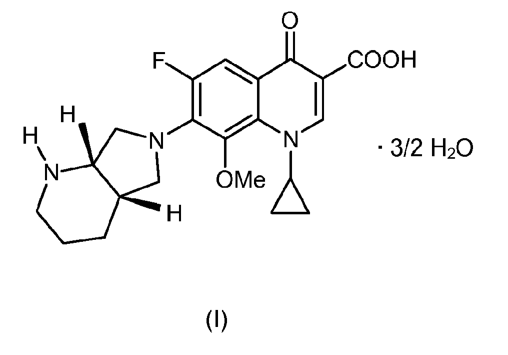

## Description

The present invention relates to a crystalline form of moxifloxacin base, to a process for its preparation, to its use as an antibacterial agent, and to pharmaceutical compositions comprising it.

### BACKGROUND ART

Moxifloxacin is a therapeutic agent that shows a broad spectrum antibacterial action. Moxifloxacin is the international non-proprietary name (INN) for 1-cyclopropyl-6-fluoro-1,4-dihydro-8-methoxy-7-[(4aS,7aS)-octahydro-6H-pyrrolo[3,4-b]pyridin-6-yl]-4-oxo-3-quinolinecarboxylic acid.

The structure of moxifloxacin corresponds to formula (I):

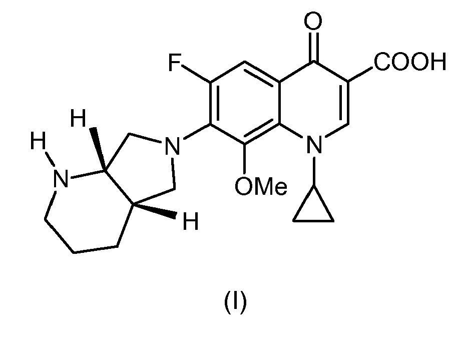

Racemic moxifloxacin was firstly described in EP-A-350733 and, particularly, moxifloxacin having a (S,S)-configuration is described in EP-A-550903.

In experimental example 19 of EP-A-550903 and example Z19 of EP-A-591808, a method for preparing and isolating moxifloxacin base is described. The same method is described in patent document EP-A-592868). These published patent applications neither describe nor suggest the possible existence of a crystalline form of moxifloxacin base. In WO-A-2008059521 it is disclosed that by performing the mentioned examples an acetonitrile solvated form of moxifloxacin with low purity is obtained, and so the obtained solvate form can not be used as such in pharmaceutical formulations. In the above mentioned examples, the obtained moxifloxacin base crude is purified and isolated by chromatography using methylene chloride/methanol/17% aqueous ammonia as the solvent system. The purification process disclosed in said documents has been reproduced by the authors of the present invention, but only an amorphous form of moxifloxacin was obtained. This process for the purification and isolation of moxifloxacin base is complex and difficult to perform on industrial scale due to the need for purifying the product by column chromatography.

WO-A-9926940 discloses a process for the preparation of moxifloxacin from a difluoro precursor comprising the step of adjusting the pH to 6.8- 7.0. However, the reproduction of this example shows that at this pH moxifloxacin hydrochloride or a mixture of moxifloxacin hydrochloride and moxifloxacin base is obtained, since the X-ray diffractogram of the isolated compound corresponds with the hydrochloride moxifloxacin. This fact has also been confirmed by the reproduction of the subsequent crystallization described in the international patent application of the previous compound in ethanol/water. The behaviour of this solid was very different regarding its solubility in respect what was described in the international patent application.

WO-A-2004091619 and WO-A-2007010555 disclose the preparation of moxifloxacin base as a solid. Nevertheless, they do not describe nor suggest the preparation of a crystalline form of moxifloxacin base. The authors of the present invention have proved that the X-ray diffractogram of the product obtained by reproducing the reference example disclosed in WO-A-2004091619, based on a pH adjustment to 7.0-7.2, corresponds to the X-ray diffractogram of the monohydrate of moxifloxacin hydrochloride disclosed in US 5849752. Similarly, by reproducing the reference example of WO-A-2007010555, also for obtaining moxifloxacin base but based on a pH adjustment to 5.0-6.0, it is neither expected to obtain moxifloxacin base due to the adjustment of the solution within a range of acidic pH values.

WO-A-2008059521 discloses a process for the preparation of a crystalline form of moxifloxacin base, designated as Form I, by recrystallization in a ketosolvent, such as acetone. This form is characterized by its X-ray diffraction pattern corresponding to a hemihydrate. The best yield obtained is a 78.2% starting from moxifloxacin hydrochloride in example 18. This crystalline form has a tendency to occlude solvent molecules within the crystalline network in amounts very superiors to the allowed ones by for Guidelines Residual Solvents (CPMP/ICH/283/95) and can be difficult to impossible to remove by drying, what forces to carry out laborious treatments, either physical, or chemical to reach allowed solvent levels. The presence of any non-aqueous solvents in amounts over the allowed ones would not make this crystalline form suitable for the preparation of pharmaceutical formulations.

The existence of polymorphs is unpredictable and there is no a priori established procedure to prepare an unknown polymorph. The difference in the physical properties of different morphological forms results from the orientation and intermolecular interactions of adjacent molecules are complexes in the bulk solid. Furthermore, the different solid forms of a pharmaceutically active ingredient can have different characteristics, and offer certain advantages in methods of manufacture and also in pharmacology. Thus, the discovery of new solid forms can contribute to clear improvements in the efficiency of methods of production and/or improvements in the characteristics of the pharmaceutical formulations of the active ingredients, since some forms are more adequate for one type of formulation, and other forms for other different formulations.

Thus, there is still a need to find new solid forms of moxifloxacin base suitable to operate on industrial scale, either to be used directly as pharmaceutical active ingredient in a pharmaceutical formulation, or if desired, to be subsequently transformed into a pharmaceutically acceptable salt thereof.

### SUMMARY OF THE INVENTION

Inventors have found a crystalline form of moxifloxacin base with improved physical properties, which is stable and pure enough to be used in the preparation of pharmaceutical compositions. This crystalline form is easy to handle, and consistently reproducible. Thus, unlike of the moxifloxacin base hemihydrate known in the art, the crystalline form of the present invention does not occlude organic solvents in its structure.

The improved physical properties of said crystalline form compared with other crystalline forms of moxifloxacin base described in the art involve enhanced manufacturing capabilities and the provision of an improved compound suitable for use in the preparation of pharmaceutical formulations.

Therefore, it is a significant contribution to the art to provide a new crystalline form of moxifloxacin base which does not occlude solvent and has the ability to crystallize and to filter easily, methods for its preparation, its use as a therapeutically active agent and pharmaceutical compositions comprising it.

For the avoidance of doubt, as used herein the term moxifloxacin, moxifloxacin base, or moxifloxacin free base is used indistinctly to address to the compound 1-cyclopropyl-6-fluoro-1,4-dihydro-8-methoxy-7-[(4aS,7aS)-octahydro-6H-pyrrolo[3,4-b]pyridin-6-yl]-4-oxo-3-quinolinecarboxylic acid of formula (I) below.

Thus, according to one aspect of the present invention, it is provided a crystalline form of moxifloxacin base of formula (I) 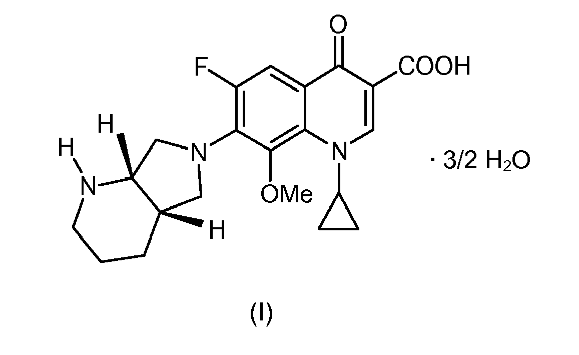 characterized by having a powder X-ray diffractogram that comprises characteristic peaks at approximately 9,6, 10,3, 12,5, 14,6, 15,2, 15,5, 16,7, 17,4, 18,1, 19,2, 21,3, 23,0, 23,4, 23,8, 24,7, 26,8, 29,4, 32,2 degrees 2 theta.

Another aspect of the invention relates to a process for the preparation of the crystalline form of moxifloxacin base as defined above, characterized by comprising the following steps: a) dissolving a suspension of moxifloxacin in an organic solvent which is able to be removed from water, by means of heating to the reflux temperature; b) adding water and cooling to a temperature ranging between 20°C to 40°C; c) removing the organic solvent; d) seeding with moxifloxacin base sesquihydrate; and e) isolating the crystalline form obtained.

Another aspect of the present invention relates to a pharmaceutical composition that comprises as active ingredient a therapeutically effective amount of the crystalline form of moxifloxacin as defined above, together with appropriate pharmaceutically acceptable excipients or carriers.

Finally, another aspect of the present invention relates to the use of the crystalline form of moxifloxacin base as defined above for the preparation of a medicament for treating diseases caused by bacteria. This aspect can also be formulated as crystalline form of moxifloxacin base as defined above for use in the treatment of diseases caused by bacteria.

The invention is also related to a method of treatment of a mammal, including a human, suffering from a disease caused by bacteria, said method comprising the administration to said patient of a therapeutically effective amount of the crystalline form of moxifloxacin base of the present invention, together with pharmaceutically acceptable excipients or carriers.

### BRIEF DESCRIPTION OF THE DRAWINGS

FIG. 1 shows the powder X-ray diffraction (PXRD) pattern of the crystalline form of moxifloxacin base of the present invention. The ordinate represents the number of counts and the abscissa the peak position at 2θ angles.
FIG. 2 shows the molecular structure (for non-hydrogen atoms) with atomic numeration.

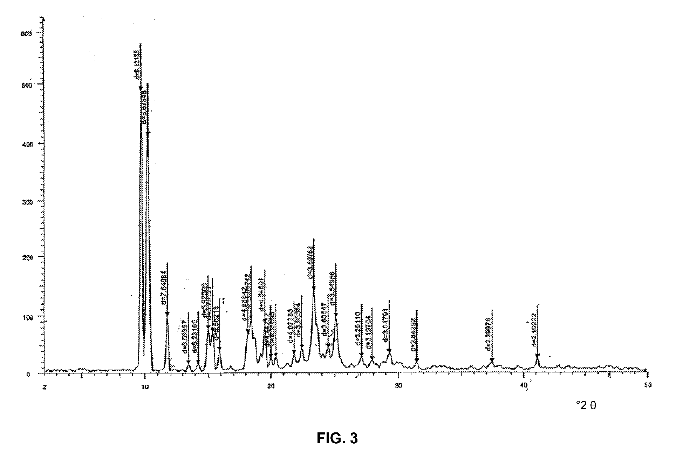

FIG. 3 shows the powder X-ray diffractogram for the Form I disclosed in WO-A-2008059521 (for comparative purposes). The ordinate represents the number of counts and the abscissa represents the peak position at 2θ angles.
FIG. 4 shows the ¹³C solid state NMR spectrum of the crystalline form of the invention.
FIG. 5 shows the thermogravimetric analysis performed with the crystalline form of the invention. The abscissa indicates the temperature (°C) and the ordinate indicates the percent mass variation in weight.

### DETAILED DESCRIPTION OF THE INVENTION

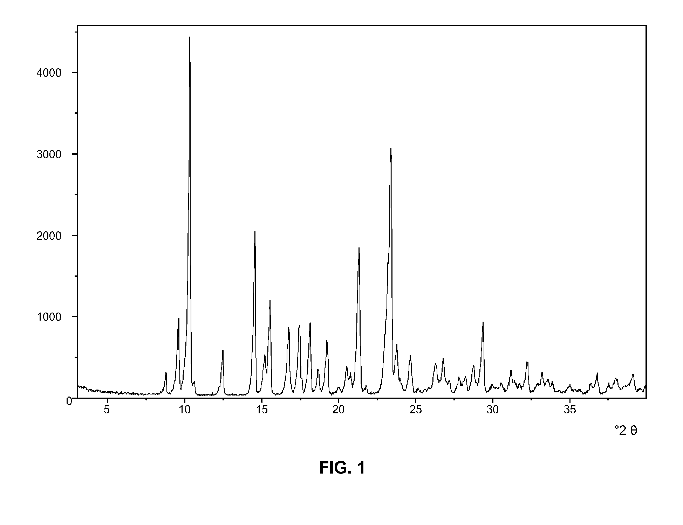

As mentioned above, the new crystalline form of moxifloxacin base of the present invention is characterized by a powder X-ray diffraction pattern, as shown in FIG. 1, comprising characteristic peaks at 2θ angles as shown in Table 1 below.

Table 1 shows the characterizing interplanar d-spacings and relative intensities of the new crystalline form of moxifloxacin base.

**Table 1**

| *X-Ray Diffraction Peaks* | | |
|---|---|---|
| 2θ (°) | d (Å) | Relative intensity (%) |
| 8,8 | 10,08 | 6,35 |
| 9,6 | 9,23 | 21,43 |
| 10,3 | 8,57 | 100 |
| 10,6 | 8,35 | 3,78 |
| 12,5 | 7,10 | 12,81 |
| 14,6 | 6,09 | 45,38 |
| 15,2 | 5,82 | 11,29 |
| 15,5 | 5,71 | 25,77 |
| 16,7 | 5,30 | 18,73 |
| 17,4 | 5,08 | 19,2 |
| 18,1 | 4,89 | 20,23 |
| 18,7 | 4,75 | 6,87 |
| 19,2 | 4,61 | 15,52 |
| 20,0 | 4,44 | 2,05 |
| 20,5 | 4,33 | 8,06 |
| 20,8 | 4,28 | 6,26 |
| 21,3 | 4,17 | 41,83 |
| 21,8 | 4,08 | 2,26 |
| 23,0 | 3,87 | 16,87 |
| 23,4 | 3,80 | 69,95 |
| 23,8 | 3,74 | 14,37 |
| 24,7 | 3,61 | 11,28 |
| 25,1 | 3,54 | 1,93 |
| 26,3 | 3,39 | 8,95 |
| 26,8 | 3,33 | 9,29 |
| 27,2 | 3,28 | 3,87 |
| 27,8 | 3,21 | 5,1 |
| 28,2 | 3,16 | 5,34 |
| 28,8 | 3,10 | 8,53 |
| 29,4 | 3,04 | 20,12 |
| 30,0 | 2,98 | 2,86 |
| 30,6 | 2,93 | 3,49 |
| 31,2 | 2,87 | 7,06 |
| 31,7 | 2,82 | 3,09 |
| 32,2 | 2,78 | 9,42 |
| 32,8 | 2,73 | 2,67 |
| 33,2 | 2,70 | 6,51 |
| 33,6 | 2,67 | 4,11 |
| 33,9 | 2,65 | 3,5 |
| 35,0 | 2,56 | 2,89 |
| 36,4 | 2,47 | 3,26 |
| 36,8 | 2,44 | 5,59 |
| 37,5 | 2,40 | 3,31 |
| 38,0 | 2,37 | 4,75 |
| 39,1 | 2,30 | 5,95 |

For recording the powder X-ray diffractogram, an X'Pert to PHILIPS automatic diffractometer was used with a Cu tube and a graphite secondary monochromator of the following specifications:
- Cu tube at 50 kV and 40 mA; X Celerator Detector; Cu_{Kα1} radiation (λ=1.5419 A)
- Receiving slit: 0.1 mm
- Soller: 0.04 radians
- Dispersion slit and divergence slit: 0.5°
- Scanning angle: 3-40° with pass of 0.03° and 1-second pass time
- HighScore software was used for automatic analysis.

This new crystalline form of moxifloxacin exhibits the following data of the monocrystal cell obtained by single crystal X-ray diffraction (SCXRD):
Crystal system Triclinic
   Space group *P1* (No. 1 in the TIC)
   Unit cell dimensions:
      a = 10.2460(6) Å α = 100.067(1)°
      b = 10.6696(6) Å β = 112.034(1)°
      c = 11.6749(6) Å γ = 112.021(1)°
   Volume= 1020.2 (1) Å³
   Density= 1.395 g/cm³

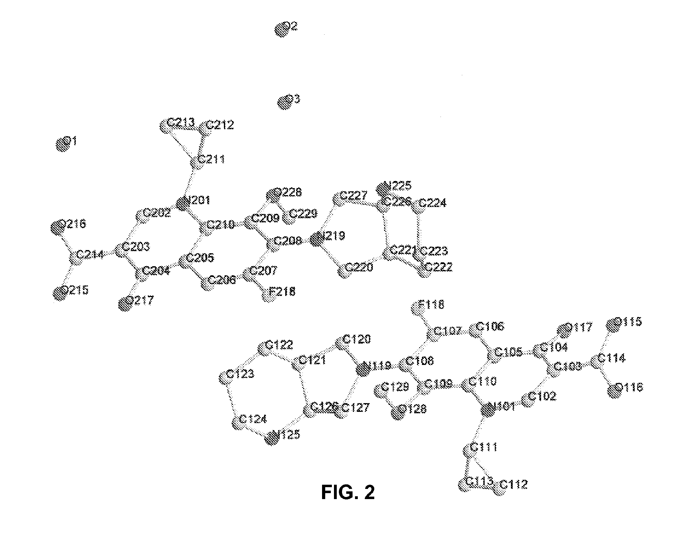

The representation of the disposition of the two independent molecules present in the elementary cell is shown in FIG. 2.

Crystal structure determination was carried out using a Bruker SMART-APEX automatic diffractometer equipped with a CCD area detector, and fitted with graphite-monochromator and Mo Kα radiation (λ = 0.71073 A).

This data confirm that the new crystalline form does not content any organic solvent in its crystalline structure, therefore it is not an organic solvate.

The crystalline form of moxifloxacin of the present invention is also characterized by ¹³C solid state nuclear magnetic resonance (¹³C NMR). The spectrum was measured on a Bruker Advance DSX300 spectrometer at 75.488 MHz using the following experimental conditions:
- Probe CP/MAS 4 mm
- Room temperature, around 23 °C
- Spinning rate around 62600 Hz
- Standard cross polarization sequence, contact time 2 ms
- Scans number 19072
- Recycling delay 3s
- Spectral width 50 KHz, time domain 12K
- Processing: SI= 16K, lorentzian apodization with line broadening 3 Hz, FT and manual phasing and baseline correction

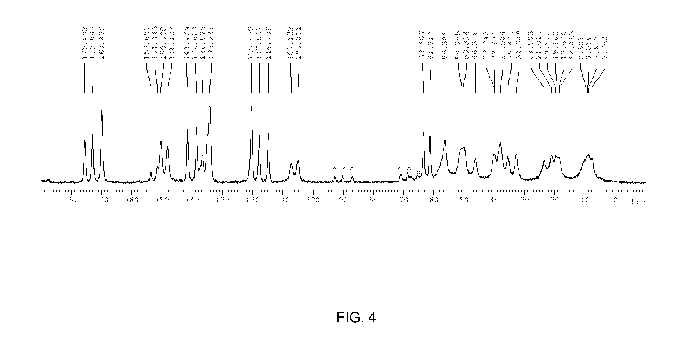

The ¹³C NMR is shown in FIG. 4 and it has the chemical displacements shown in Table 2 below.

**Table 2**

| *Chemical displacements in 13C solid state NMR spectra* |
|---|
| δ (ppm) |
| 7.77 |
| 8.82 |
| 9.05 |
| 9.68 |
| 18.47 |
| 18.67 |
| 19.14 |
| 19.57 |
| 21.01 |
| 23.58 |
| 32.65 |
| 35.48 |
| 37.90 |
| 39.79 |
| 39.94 |
| 46.32 |
| 50.33 |
| 50.70 |
| 56.31 |
| 61.32 |
| 63.41 |
| 105.01 |
| 107.12 |
| 114.74 |
| 117.83 |
| 120.44 |
| 134.24 |
| 136.53 |
| 138.60 |
| 141.43 |
| 148.14 |
| 150.30 |
| 151.45 |
| 153.65 |
| 169.82 |
| 172.95 |
| 175.49 |

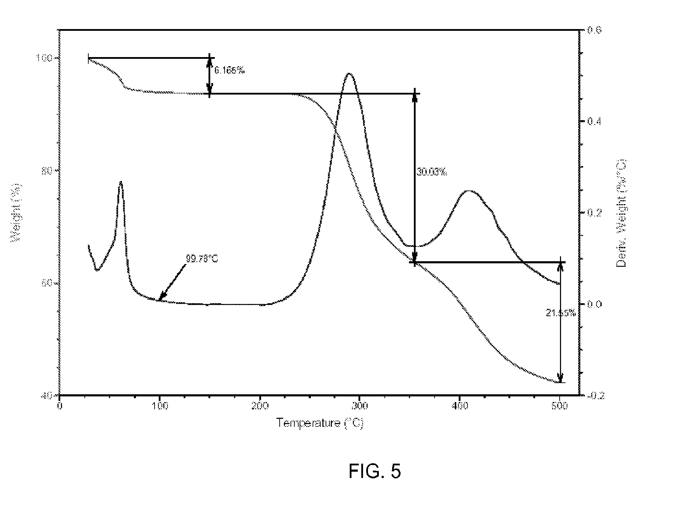

The crystalline form of moxifloxacin of the present invention is also characterized by thermogravimetry (TG) analysis. The results of the TG analysis are shown in FIG. 5. A TGA Q500 V6.5 analyzer was used to perform the termogravimetric analysis, heating up to 520° C (10° C./min), N₂ (90 mL/min), platinum-made sample pan.

The TG curve shows a two-stage mass loss. The first mass loss of about 6.2 % takes place at a temperature range of 50-150 °C. The sample is stable until a temperature of about 225 °C, and then a continuous loss begins at a rate of approximately 30 % at 335 °C and about 21 % at 500 °C.

As mentioned above, the crystalline form of moxifloxacin of the present invention can be prepared by a process comprising the following steps:
a) dissolving a suspension of moxifloxacin in an organic solvent which is able to be removed from water, by means of heating to the reflux temperature;
b) adding water and cooling to a temperature ranging between 20°C to 40°C;
c) removing the organic solvent;
d) seeding with moxifloxacin base sesquihydrate; and
e) isolating the crystalline form obtained.

Moxifloxacin used as starting material may be prepared by any of the processes described in the prior art.

By the expression "an organic solvent which is able to be removed from water" it is understood that it can be separated from water, for instance by distillation, remaining the rest of the components of the starting solution in the aqueous phase.

Preferably, the organic solvent used in step a) is a hydroxylic solvent or a solvent miscible with water. Example of appropriate hydroxylic solvents include (C₁-C₄)-alcohols such as methanol, ethanol, isopropanol, butanol or, tert-butanol, or a glycol such as ethylenglycol or propylenglycol. Examples of appropriate water miscible solvents are acetonitrile, acetone, N,N-dimethylformamide, dimethylsulfoxide or N-methylpirrolidone. More preferably, the organic solvent used in step a) is acetonitrile, ethanol or methanol. Most preferably, it is acetonitrile.

Generally, the starting moxifloxacin is dissolved in the organic solvent, such as acetonitrile, by heating to reflux temperature. Water is added and is cooled to a temperature between 20°C and 40°C. Generally, the amount of water is comprised between 5-10 ml per g of moxifloxacin base. Then, the organic solvent is removed by distillation before crystallization is performed. Generally, the amount of the remaining water is comprised between 2-4 ml per g of moxifloxacin base. It is seeded with moxifloxacin base sesquihydrate, and it is allowed to stand until crystallization of the product. Generally, the crystallization occurs at a temperature comprised between 15-20 °C.

The moxifloxacin base sesquihydrate which is used for seeding for the first time is prepared by means of the process described in the Preparative Example set out below in this description.

The separation of the product from the supernatant solution can be done by a conventional method such as filtration. The solid filters readily, thereby making easy the preparation.

The remaining solvent can be removed from the product by drying, optionally under vacuum. The product is also easily dried, since it does not occlude solvent into its crystalline structure.

The new crystalline for of moxifloxacin base obtainable by this process has a water content ranging 6,1 to 6,5% w/w and remains stable for at least 9 months in contact with the atmosphere in environmental conditions. The water content corresponds with a sesquihydrate which has a theoretical water content of 6,3%.

Thus, the physical properties of the crystalline form of moxifloxacin allow for good manipulation during all the preparation process. Furthermore, the process is reproducible, simple and suitable for industrial scale.

The new crystalline form of the present invention can be converted into a pharmaceutically acceptable salt by methods known in the art, for instance, by reaction with the corresponding pharmaceutically acceptable acid in an appropriate solvent or by ions exchange between a salt of moxifloxacin and an inorganic or organic salt. Preferably, the pharmaceutically acceptable salt is the hydrochloride.

The most adequate conditions for carrying out said processes vary depending on the parameters considered by the expert in the art, such as, for example, the concentration of the starting material, temperature, and the like. These can be easily determined by said skilled person in the art by routine tests and with the help of the teachings of the examples given in this description.

The pharmaceutical composition of the present invention comprises a therapeutically effective amount of the crystalline form of moxifloxacin, together with suitable pharmaceutically acceptable excipients or carriers. The compound of the present invention can be normally formulated in accordance with standard pharmaceutical practice.

The crystalline form of moxifloxacin of the present invention is useful in the treatment of diseases caused by bacteria.

Throughout the description and the claims the word "comprises" and its variants are not meant to exclude other technical characteristics, additives, components or steps. For skilled persons in the art, other objects, advantages and characteristics of the invention can be deduced in part from the description and partly from the practice of the invention. The following examples are provided for illustrative means, and are not meant to be limiting of the present invention.

### EXAMPLES

### Preparative example: Preparation of monocrystals of moxifloxacin base sesquihydrate for seeding

A suspension of 10 g of moxifloxacin free base in 90 ml of H₂O was heated at a temperature of 80°C for 15 minutes under stirring. Then the resulting suspension was cooled down between 20 and 30 °C, and the insoluble solid was filtered off followed by washing with 10 ml of water. Then, the wash waters and mother liquor were combined and allowed to stand at room temperature in a closed flask for about 20 days until complete crystallization of the product. Finally, the crystalline solid was filtered, washed with 2 ml of water and dried under vacuum until constant humidity.

The analysis of the new crystalline form was carried out by single crystal X-ray diffraction. The parameters obtained allowed to identify the sesquihydrate of the new crystalline moxifloxacin base.

The product obtained is used for the seeding in example 1.

### Example 1: Preparation of crystalline of moxifloxacin base sesquihydrate

A suspension of 5 g of moxifloxacin base in 85 ml of acetonitrile was refluxed until complete dissolution. To the resulting solution, 30 ml of water were added and it was cooled at 20-40 °C. Then, part of the solvent was distilled off from the solution until a volume of about 20 ml to completely remove acetonitrile, and the resulting aqueous solution was seeded with moxifloxacin base sesquihydrate to begin the crystallization. The mixture was stirred at 15-20°C for about 20 hours and, then, the crystals were collected by filtration and washed with 5 ml of water. The solid was dried under reduced pressure at room temperature to obtain 4.89 g (91.6% yield) of moxifloxacin base sesquihydrate as a white solid.

## Claims

1. A crystalline form of moxifloxacin base of formula (I), 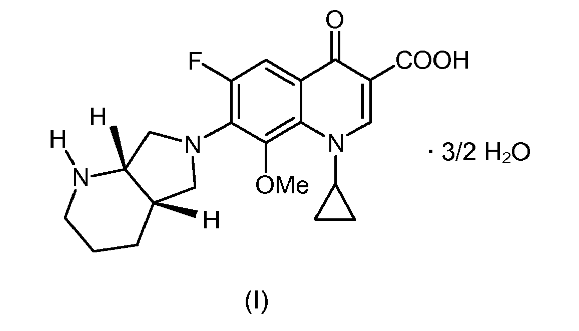 **characterized by** having an X-ray diffraction pattern that comprises characteristic peaks at approximately 9,6, 10,3, 12,5, 14,6, 15,2, 15,5, 16,7, 17,4, 18,1, 19,2, 21,3, 23,0, 23,4, 23,8, 24,7, 26,8, 29,4, 32,2 degrees 2 theta.

2. The crystalline form of moxifloxacin according to claim 1, further **characterized by** having substantially the same X-ray diffraction pattern as shown in FIG 1.

3. The crystalline form of moxifloxacin base according to any one of claims 1-2, **characterized by** having the ¹³C solid-state NMR spectrum as shown in FIG. 4.

4. A process for the preparation of the crystalline form of moxifloxacin base according to any one of claims 1 to 3, **characterized by** comprising the following steps:
a) dissolving a suspension of moxifloxacin in an organic solvent which is able to be removed from water, by means of heating to the reflux temperature;
b) adding water and cooling to a temperature ranging between 20°C to 40°C;
c) removing the organic solvent;
d) seeding with moxifloxacin base sesquihydrate; and
e) isolating the crystalline form obtained.

5. The process according to claim 4, wherein the organic solvent of step a) is selected from the group consisting of acetonitrile, ethanol, and methanol.

6. The process according to claim 5, wherein the organic solvent is acetonitrile.

7. A pharmaceutical composition comprising a therapeutically effective amount of the crystalline form of moxifloxacin as defined in any one of claims 1 to 3, together with appropriate amounts of pharmaceutical excipients or carriers.

8. Use of the crystalline form of moxifloxacin base as defined in any one of claims 1 to 3, for the preparation of a medicament for treating diseases caused by bacteria.

9. Crystalline form of moxifloxacin base as defined in any one of claims 1 to 3, for use in the treatment of diseases caused by bacteria.
